# EUROPEAN PATENT APPLICATION

(11) **EP 4 194 547 A1**
(43) Date of publication of application: **14.06.2023**
(21) Application number: 21852621.8
(22) Date of filing: 28.06.2021
(51) Int. Cl.: C12N 5/00, C12M 3/00, G01N 33/569

(54) **METHOD FOR SCREENING FOR TARGET CELLS OR CELLS, AND BIOLOGICAL CULTURE CHIP**

(30) Priority: 04.08.2020 CN 202010771818; 04.08.2020 CN 202010771819; 04.08.2020 CN 202010771453
(71) Applicant: Nanjing Livingchip Biotechnology Co., Ltd., Nanjing, Jiangsu 211100 (CN)
(72) Inventor: GAO, Xiaohang, Nanjing, Jiangsu 211100 (CN); WEI, Yu, Nanjing, Jiangsu 211100 (CN)
(74) Representative: Petraz, Gilberto Luigi
(86) International application number: PCT/CN2021/102799
(87) International publication number: WO 2022/028150

(57) **Abstract**

A method for screening for target cells, a corresponding test kit and a use thereof, a method for screening cells, and a biological culture chip and a preparation method therefor and a use thereof. The method for screening target cells comprises: culturing said candidate single cells within a culture chamber provided with a signal screening layer, the signal screening layer comprising signal molecules for specific recognition of target molecules; on the basis of signals of the signal molecules, selecting target cells suitable for secreting the target molecules. The method for screening cells comprises: arranging candidate cells within a culture chamber to form target antibody-antigen-signal antibody compounds; on the basis of signals of signal molecules connected to the signal antibodies, determining whether the candidate cells are target cells. The biological culture chip comprises a matrix (100), and a biological culture space arranged on the surface of the matrix (100) and used for culturing biological cells.

## Description

### TECHNICAL FIELD

The present disclosure relates to the field of biotechnology, and more particularly, to a method for screening target cells, a kit and its use thereof according to a first aspect of the present disclosure, to a method for screening target cells, a kit, a method for preparing antibodies, a method for screening for a monoclonal antibody, a monoclonal antibody, an antibody-antigen complex, and a method for screening antibodies according to a second aspect of the present disclosure, and to a biological culture chip, a preparation method and use thereof according to a third aspect of the present disclosure.

### BACKGROUND

Limiting dilution is a commonly used cloning method and is achieved by pipetting a cell line to be re-cloned out of culture wells and measuring the cells of 1 mL. This method is commonly used for screening fused animal cells. A monoclonal antibody is a highly homogeneous antibody, only targeting a specific epitope, produced by cloning a single B cell. Monoclonal antibodies are usually produced by hybridoma technology. The hybridoma technology is based on cell fusion. The sensitized B cells with the capacity of secreting specific antibodies and the myeloma cells with the capacity of unlimited reproduction are fused into a B-cell hybridoma.

However, existing methods for screening cells that can express target molecules, especially fused cells for monoclonal antibodies, still need to be improved.

Antibody pairs are widely used in protein quantitation. Sandwich ELISA, chemiluminescence, and immunochromatography cannot be conducted without high-quality antibody pairs. An antibody pair is a set of two antibodies that can simultaneously bind to one antigen molecule. The antibody pairs are usually used in double-antibody sandwich ELISA experiments. Generally, screening for the antibody pairs is carried out by a double-antibody sandwich ELISA method. The screening is based on the following description. After monoclonal antibodies are obtained, two types of monoclonal antibodies are separated therefrom, one as a capture antibody, and the other as a labeled antibody (HRP enzyme labeling). The capture antibodies are coated on an antigen capture plate. First, antigens are added and incubated, and the unbound antigens are washed away. Then, the labeled antibodies are added and incubated, and the unbound labeled antibodies are washed away. Finally, a color developing solution is added for developing the color. On a condition of the color can be developed, it indicates that the labeled antibodies specifically bind to the antigens, and the capture antibody and the labeled antibody form an antibody pair. On a condition of the color cannot be developed, it indicates that the labeled antibodies cannot specifically bind to the antigens and are washed away, and the capture antibody and the labeled antibody cannot form an antibody pair. On a condition of the two types of selected monoclonal antibodies cannot be paired, it is necessary to reselect two types of monoclonal antibodies for a retest, until an antibody pair is found.

However, the current antibody pair screening efficiency is very low efficiency, resulting in a substantial increase in screening costs.

In the current biological and medical research, it is preferred to carry out cytological tests on drugs for detection, by conventional two-dimensional (2D) adherent cell culture in most cases. This is essentially different from the existence state (a three-dimensional (3D) state in an ECM environment) of real tissue cells *in vivo.* Cells in a living organism grow in a 3D microenvironment, while cells that grow by 2D culture are not in the natural state of cell growth. The culture microenvironment is quite different from the *in vivo* microenvironment, which affects gene expression and signal transduction of cells, causing the cultured cells to gradually lose their biological characteristics and functions in the living organism, and lose their research and application value. There are also many significant defects in 2D cell culture, such as uneven differentiation of cells, which is not conducive to the cell differentiation research and related research. In addition, there are a large number of cells obtained from 2D culture, but it is difficult to sort useful cells out of these cells, and a waste of reagents is caused.

CN108102913A discloses a 3D cell culture chip based on soft lithography, a preparation method and use thereof. The preparation method of the 3D cell culture chip based on soft lithography includes: producing a mask with a set pattern structure by soft lithography; uniformly covering the mask with a liquid polymer compound or polymer compound solution to form a liquid layer with a set thickness; solidifying the liquid layer, and removing the mask, to obtain a stamp with a stamp surface having a set 3D structure; bringing the stamp surface of the stamp into contact with a modifier, so that the modifier is attached to the stamp surface detachably; and bringing the stamp surface into contact with a surface of a selected substrate, and removing the stamp from the selected substrate, so that at least a part of the modifier is separated from the stamp surface and attached to the surface of the selected substrate to form a pattern structure including an array consisting of a plurality of patterns that can at least adsorb single cells, to obtain the 3D cell culture chip.

However, existing chips for the culture of biocompatible materials still need to be further improved, which needs not only be suitable for the 2D array and 3D culture of single cells, but also be suitable for complex detection of cell biology functions, such as antibody secretion, growth factor secretion and so on.

### SUMMARY

The present disclosure aims to resolve at least one of the technical problems existing in the prior art. Therefore, according to an aspect of the present disclosure, a method for screening target cells is provided, the target cells being suitable for secreting target molecules, the method comprising: culturing candidate single cells in a culture chamber under a condition suitable for secreting the target molecules, wherein the culture chamber includes a signal screening layer for covering the candidate single cells, and wherein the signal screening layer comprises signaling molecules for specific recognition of the target molecules; and selecting the target cells suitable for secreting the target molecules based on signals of the signaling molecules in the signal screening layer. By this method, since only single cells exist in the culture chamber, on a condition of the single cells can secrete target molecules during culture, the target molecules can be specifically captured by the signaling molecules. Based on this, the signaling molecules can be aggregated within a specific range to amplify the signals of the signaling molecules. Therefore, whether the single cells can express the target molecules can be determined based on signal difference of the signaling molecules. Compared with limiting dilution in the related art, this method is fast, cost-effective, and efficient, and can achieve high-throughput screening.

According to a second aspect of the present disclosure, the present disclosure provides a kit, comprising: a hydrogel; a signaling molecule; and a biological culture chip. By using the kit, the method above can be effectively implemented, thereby achieving the effect above. In addition, the features described above for the method are also suitable for the kit, and details are not described herein.

According to another aspect of the present disclosure, a method for preparing antibodies is further provided, which comprising: screening target cells by the method above; and allowing the target cells to proliferate and express antibodies. Therefore, fused cells that can express a monoclonal antibody can be effectively obtained.

According to another aspect of the present disclosure, a method for screening a monoclonal antibody is further provided, comprising: dispensing a plurality of hybridoma cells onto the biological culture chip, wherein each culture chamber contains at most one of the hybridoma cells; selecting positive hybridoma cells by the method above; isolating the positive hybridoma cells; and culturing the positive hybridoma cells to express antibodies, to obtain the monoclonal antibody. As described above, by the method of the present disclosure, fused cells that express a monoclonal antibody can be effectively obtained, and the monoclonal antibody can be further obtained.

According to another aspect of the present disclosure, a monoclonal antibody is further provided, obtained by the method above.

According to another aspect of the present disclosure, an antibody-antigen complex is further provided. The antibody is the monoclonal antibody above, and the monoclonal antibody is linked to a signaling molecule. By using the antibody-antigen complex, paired antibodies can be effectively obtained, one of which is an antibody with a different antigenic determinant from the known monoclonal antibody.

According to another aspect of the present disclosure, a method for screening antibodies is further provided, comprising: (1) arranging candidate cells within a culture chamber to be cultured for a predetermined time under a condition suitable for candidate cells to express antibodies; (2) adding a signaling antibody-antigen complex into the culture chamber, wherein the signaling antibody is linked to a signaling molecule, and the signaling antibody is the monoclonal antibody above; and (3) determining whether the candidate cells are target cells based on signals of the signaling molecules, wherein the target cells secrete antibodies with different antigenic determinants from the monoclonal antibody.

According to another aspect of the present disclosure, a method for screening cells is provided, comprising: (1) placing candidate cells in a culture chamber for a predetermined time under a condition suitable for candidate cells to express antibodies, the candidate cells within a culture chamber to be cultured for a predetermined time; (2) forming a target antibody-antigen-signaling antibody complex in the culture chamber, wherein the signaling antibody is linked to a signaling molecule; and (3) determining whether the candidate cells are target cells based on signals of the signaling molecules. By the method, an antibody pair can be effectively obtained by screening.

According to an embodiment of the present disclosure, a kit is provided, comprising:
a signaling antibody;
an antigen; and
a biological culture chip.

According to an embodiment of the present disclosure, a method for preparing antibodies is provided, comprising:
screening for target cells by the method mentioned above; and
allowing the target cells to proliferate and express antibodies to obtain the antibodies with different antigenic determinants from the signaling antibodies.

According to an embodiment of the present disclosure, a method for screening a monoclonal antibody is provided, comprising:
dispensing a plurality of hybridoma cells onto the biological culture chip, wherein each culture chamber contains at most one of the hybridoma cells;
selecting positive hybridoma cells by the method above;
isolating the positive hybridoma cells; and
culturing the positive hybridoma cells to express antibodies, to obtain the monoclonal antibody with a different antigenic determinant from the signaling antibody.

The present disclosure further provides a monoclonal antibody obtained by the method above. The monoclonal antibody and the signaling antibody correspond to different antigenic determinants.

According to another aspect of the present disclosure, a biological culture chip is provided, comprising: a matrix, formed from the matrix material, wherein an equilibrium swelling ratio of the matrix material of the matrix is approximately 1.25-1.75; and the culture chamber formed on a surface of the matrix to define the culture chamber.

As the matrix material has a certain equilibrium swelling ratio, the entire surface of a biocompatible material, for example, a single cell, cultured by using the biological culture chip can be surrounded by a relatively uniform liquid or gel medium. Therefore, during cell culture, the biological activity of cells can further improved to facilitate the realization of cell biology functions (such as antibodies and factors) and facilitate accurate detection.

The additional aspects and advantages of the present disclosure will be provided in the following description, some of which will become apparent from the following description or may be learned from practices of the present disclosure.

### BRIEF DESCRIPTION OF THE DRAWINGS

The above and/or additional aspects and advantages of the present disclosure will become apparent and comprehensible from the following descriptions of embodiments with reference to the following accompanying drawings.
FIG. 1 is a schematic structural diagram of a biological culture chip according to an embodiment of the present disclosure;
FIG. 2 is a schematic structural diagram of a biological culture chip according to another embodiment of the present disclosure;
FIG. 3 is a schematic structural diagram of a biological culture chip according to another embodiment of the present disclosure;
FIG. 4 is a schematic structural diagram of a template for preparing a biological culture chip according to an embodiment of the present disclosure;
FIG. 5 shows a method for screening for a monoclonal antibody according to an embodiment of the present disclosure;
FIG. 6 shows a method for screening for an antibody pair according to an embodiment of the present disclosure; and

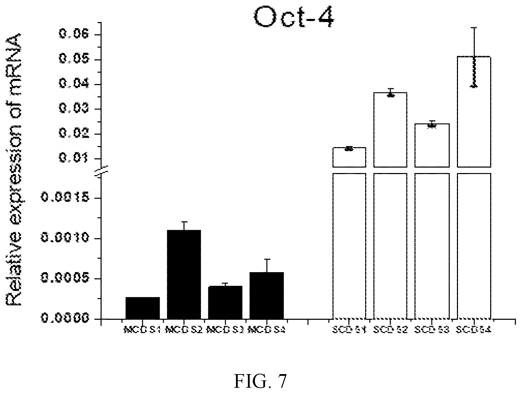

FIG. 7 shows comparison of sternness of neural stem cells between suspension culture and chip culture according to an embodiment of the present disclosure.

### DETAILED DESCRIPTION OF THE EMBODIMENTS

The embodiments of the present disclosure are described in detail below, and examples of the embodiments are shown in the accompanying drawings, where the same or similar elements or the elements having same or similar functions are denoted by the same or similar reference numerals throughout the description. The embodiments described below with reference to the accompanying drawings are exemplary and used only for explaining the present disclosure, and should not be construed as a limitation on the present disclosure.

### Method for Screening for Target Cells

According to an aspect of the present disclosure, a method for screening target cells is provided, the target cells being suitable for secreting target molecules, the method including: culturing candidate single cells in a culture chamber under a condition suitable for secreting the target molecules, wherein the culture chamber includes a signal screening layer for covering the candidate single cells, and wherein the signal screening layer comprises signaling molecules for specific recognition of the target molecules; and selecting the target cells suitable for secreting the target molecules based on signals of the signaling molecules in the signal screening layer. By this method, since only single cells exist in the culture chamber, on a condition of the single cells can secrete target molecules during culture, the target molecules can be specifically captured by the signaling molecules. Based on this, the signaling molecules can be aggregated within a specific range to amplify the signals of the signaling molecules. Therefore, whether the single cells can express the target molecules can be determined based on signal difference of the signaling molecules. Compared with limiting dilution in the related art, this method is fast, cost-effective, and efficient, and can achieve high-throughput screening.

According to an embodiment of the present disclosure, the signal screening layer is arranged in a matrix material of the culture chamber; the signal screening layer is arranged in a culture medium; the signal screening layer comprises at least one kind of antibodies or probes; the signal screening layer is formed from a hydrogel; or a mechanical strength of the matrix material is higher than a mechanical strength of the signal screening layer. Therefore, this facilitates the molecules secreted by the single cells to enter the signal screening layer, thereby increasing the screening efficiency.

According to an embodiment of the present disclosure, the culture chamber is arranged on a biological culture chip, which comprises: a matrix formed from the matrix material; and the culture chamber formed on a surface of the matrix to define the culture chamber. The biological culture chip is briefly described herein and will be described in detail below. In the process of in-depth research on 3D cell culture chips, it is found that it is usually difficult for cells to exhibit biological and physiological behaviors in the *in vivo* environment during the culture of cells or biocompatible materials by using the existing 3D culture chips. To this end, it is found through creative research that the matrix material of the existing 3D culture chip affects the effect of cell culture, especially having a significant negative impact on the realization of biology functions of single-cell secreted factors (including antibodies). Further, it is found that in the existing 3D culture chip, different positions of contact between the cell and the matrix are exposed to significantly different surrounding culture environments, and the obtained microenvironments of the cell at different positions also increase the difference and heterogeneity. For example, at a position of contact between the cell and the matrix, the cell cannot have access to sufficient support from the extracellular matrix. In addition, most of the existing 3D culture chips adopt a hard matrix. Therefore, during the process of cell culture, the hard matrix exerts a certain stress on the cultured cells, affecting the biological behavior of the cells. These cases seriously affect the capacity of factor or antibody secretion of single cells and the functional detection.

According to an embodiment of the present disclosure, the signaling molecules are fluorescent molecules.

According to an embodiment of the present disclosure, the fluorescent molecules include at least one of FITC or AF488.

According to an embodiment of the present disclosure, the method further comprises positioning positive cells by a fluorescence microscope.

According to a second aspect of the present disclosure, the present disclosure provides a kit, including: a hydrogel; a signaling molecule; and a biological culture chip. By using the kit, the method above can be effectively implemented, thereby achieving the effect above. In addition, the features described above for the method are also suitable for the kit, and details are not described herein.

According to another aspect of the present disclosure, a method for preparing antibodies is further provided, which comprising: screening target cells by the method above; and allowing the target cells to proliferate and express antibodies. Therefore, fused cells that can express a monoclonal antibody can be effectively obtained.

According to another aspect of the present disclosure, a method for screening for a monoclonal antibody is further provided, including: dispensing a plurality of hybridoma cells onto the biological culture chip, where each culture chamber contains at most one of the hybridoma cells; selecting positive hybridoma cells by the method above; isolating the positive hybridoma cells; and culturing the positive hybridoma cells to express antibodies, to obtain the monoclonal antibody. As described above, by the method of the present disclosure, fused cells that express a monoclonal antibody can be effectively obtained, and the monoclonal antibody can be further obtained.

According to another aspect of the present disclosure, a monoclonal antibody is further provided, obtained by the method above.

According to another aspect of the present disclosure, an antibody-antigen complex is further provided. The antibody is the monoclonal antibody above, and the monoclonal antibody is linked to a signaling molecule. By using the antibody-antigen complex, paired antibodies can be effectively obtained, one of which is an antibody with a different antigenic determinant from the known monoclonal antibody.

According to another aspect of the present disclosure, a method for screening antibodies is further provided, including: (1) arranging candidate cells within a culture chamber to be cultured for a predetermined time under a condition suitable for candidate cells to express antibodies; (2) adding a signaling antibody-antigen complex into the culture chamber, where the signaling antibody is linked to a signaling molecule, and the signaling antibody is the monoclonal antibody above; and (3) determining whether the candidate cells are target cells based on signals of the signaling molecules, where the target cells secrete antibodies with different antigenic determinants from the monoclonal antibody.

According to an embodiment of the present disclosure, compared with conventional limiting dilution, the method of the present disclosure has an absolute advantage in terms of screening efficiency and screening cost. Taking the screening monoclonal antibodies as an example,while there is a decrease of about 90% in the overall cost and an increase of at least 10 times in the screening efficiency. This is only the result obtained without conducting a comprehensive screening, and personnel and time can be arranged more properly according to the urgency degree of a project. Based on this, it is believed that the screening efficiency can be effectively increased by 30 times or more.

Therefore, a method for screening target cells is provided, the target cells being suitable for secreting target molecules, the method including: culturing candidate single cells in a culture chamber under a condition suitable for secreting the target molecules, wherein the culture chamber includes a signal screening layer for covering the candidate single cells, and wherein the signal screening layer comprises signaling molecules for specific recognition of the target molecules; and selecting the target cells suitable for secreting the target molecules based on signals of the signaling molecules in the signal screening layer.

A person skilled in the art can understand that the culture chamber may be a culture space in the biological culture chip described in this specification. Therefore, the advantages and features of the biological culture chip above are also suitable for this method for screening, and details are not described herein. It is to be emphasized that, according to an embodiment of the present disclosure, the signal screening layer may be used as a liquid or gel medium to cover single cells, or may be used as a solid or semi-solid matrix. According to an embodiment of the present disclosure, considering the need to isolate single cells, a gel material with a mechanical strength lower than the matrix may be selected. For example, a material with a concentration controlled to be lower than the gel material is used as the signal screening layer.

The signal screening layer contains at least one kind of labeled antibodies, proteins, polypeptides, or probes.

According to an embodiment of the present disclosure, the signal screening layer is formed from a first hydrogel, the culture chamber is provided on a biological culture chip, and
a concentration of the first hydrogel is lower than a concentration of a second hydrogel.

Therefore, according to still another aspect of the present disclosure, a kit is provided, including:
a hydrogel;
a signaling molecule; and
a biological culture chip.

By using the kit, the method above can be effectively implemented. The features and advantages about the method are also suitable for the kit, and details are not described herein.

Further, according to another aspect of the present disclosure, a method for screening antibodies is further provided, including:
screening for target cells by the method above; and
allowing the target cells to proliferate and express antibodies.

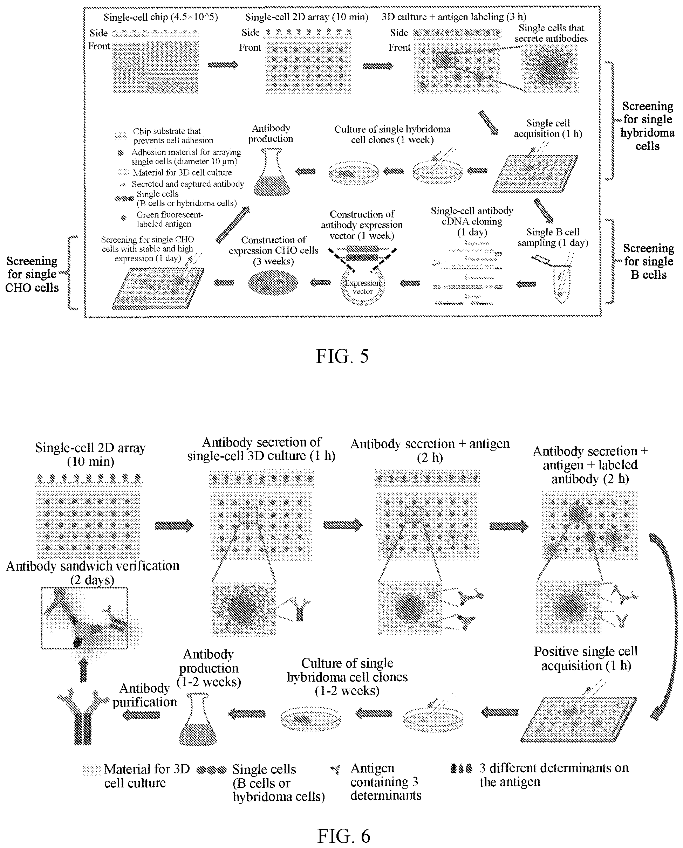

Referring to FIG. 5, the present disclosure further provides a method for screening a monoclonal antibody from single cells, including:
dispensing a plurality of hybridoma cells onto the biological culture chip to form an array, where each culture chamber in the biological culture contains at most one of the hybridoma cells;
placing the biological culture chip containing the array of the hybridoma cells in a culture medium containing a labeled target antigen (protein or polypeptide) to undergo an antigen-antibody reaction, and selecting labeled positive hybridoma cells that secrete a target antibody;
isolating the positive hybridoma cells; and
culturing the positive hybridoma cells to express antibodies, to obtain the single cell-derived monoclonal antibody.

Based on this, another aspect of the present disclosure further provides a monoclonal antibody from single cells, which is obtained by the method above.

Therefore, according to another aspect of the present disclosure, an antibody-antigen complex is further provided, where the antibody is the monoclonal antibody above, and the monoclonal antibody is linked to a signaling molecule.

Further, referring to FIG. 6, another aspect of the present disclosure further provides a method for high-throughput screening for paired antibodies against the same antigen but different antigenic determinants (paired antibodies for short), including the following steps:
(1) arranging candidate cells within a culture chamber to be cultured for a predetermined time under a condition suitable for candidate cells to express antibodies;
(2) adding a signaling antibody-antigen complex into the culture chamber, where the signaling antibody is linked to a signaling molecule, and the signaling antibody is the above monoclonal antibody from single cells or its derivatives, such as an antibody fragment binding to a specific antigenic determinant, including a fragment of antigen binding (Fab), (Fab)₂, Fv, and the like; and

an unlabeled antigen is added in the culture chamber, on a condition of the cell secretes an antibody, the secreted antibody and the antigen will form an antibody-antigen complex and the antibody-antigen complex will be captured around the cell, and then the signaling antibody is added into the culture medium in the biological culture chip, wherein the signaling antibody is linked to the signaling molecule, and the signaling antibody is the above monoclonal antibody from single cells or its derivatives, such as an antibody fragment binding to a specific antigenic determinant, including a fragment of antigen binding (Fab), (Fab)₂, Fv, and the like;
and (3) determining whether the candidate cells are target cells based on signals of the signaling molecules, where the target cells secrete antibodies with different antigenic determinants from the single cell-derived monoclonal antibody.

### Method for Screening for Antibody Pair

According to another aspect of the present disclosure, a method for screening cells is provided, including: (1) placing candidate cells in a culture chamber for a predetermined time under a condition suitable for candidate cells to express antibodies; (2) forming a target antibody-antigen-signaling antibody complex in the culture chamber, where the signaling antibody is linked to a signaling molecule; and (3) determining whether the candidate cells are target cells based on signals of the signaling molecules. By the method, an antibody pair can be effectively obtained by screening. By the method, the candidate cells are arranged in the culture chamber for single-cell culture, and the antibodies secreted by the cells form the target antibody-antigen-signaling antibody complex to enrich the signaling molecules in a certain range, thereby effectively achieving the screening for the antibody pair. Compared with limiting dilution in the related art, this method is fast, cost-effective, and efficient, and can achieve high-throughput screening.

According to an embodiment of the present disclosure, the predetermined time is 1-10 hours, preferably 2-5 hours, more preferably 3 hours.

According to an embodiment of the present disclosure, the culture chamber is configured on a biological culture chip (which will be described in detail below), and the target antibody-antigen-signaling antibody complex is formed by adding an antigen-signaling antibody complex into the culture chamber or sequentially adding a free antigen and a free signaling antibody.

According to an embodiment of the present disclosure, the signaling molecules are fluorescent molecules, preferably, the fluorescent molecules include at least one of FITC or AF488.

According to an embodiment of the present disclosure, in step (3), the method further comprises positioning positive cells based on the signals of the signaling molecules by a fluorescence microscope.

According to an embodiment of the present disclosure, a kit is provided, including:
a signaling antibody;
an antigen; and
a biological culture chip.

According to an embodiment of the present disclosure, a method for preparing antibodies is provided, including:
screening for target cells by the method above; and
allowing the target cells to proliferate and express antibodies to obtain the antibodies with different antigenic determinants from the signaling antibodies.

According to an embodiment of the present disclosure, a method for screening for a monoclonal antibody is provided, including:
dispensing a plurality of hybridoma cells onto the biological culture chip, where each culture chamber of the biological culture chip contains at most one of the hybridoma cells;
selecting positive hybridoma cells by the method above;
isolating the positive hybridoma cells; and
culturing the positive hybridoma cells to express antibodies, to obtain the monoclonal antibody with a different antigenic determinant from the signaling antibody.

According to another aspect of the present disclosure, a monoclonal antibody is further provided, obtained by the method above. The monoclonal antibody and the signaling antibody correspond to different antigenic determinants.

The signaling antibody used for screening for an antibody pair may be obtained or prepared by the following method.

A person skilled in the art can understand that the culture chamber may be a culture space in the biological culture chip described in this specification. Therefore, the advantages and features of the biological culture chip above are also suitable for this method for screening, and details are not described herein. It is to be emphasized that, according to an embodiment of the present disclosure, the signal screening layer may be used as a liquid or gel medium to cover single cells, or may be used as a solid or semi-solid matrix. According to an embodiment of the present disclosure, considering the need to isolate single cells, a gel material with a mechanical strength lower than the matrix may be selected. For example, a material with a concentration controlled to be lower than the gel material is used as the signal screening layer.

The signal screening layer contains at least one kind of labeled antibodies, proteins, polypeptides, or probes. For ease of understanding, the following describes a biological culture chip suitable for the present disclosure in detail.

### Biological Culture Chip

A biological culture chip according to an embodiment of the present disclosure is described with reference to the accompanying drawings.

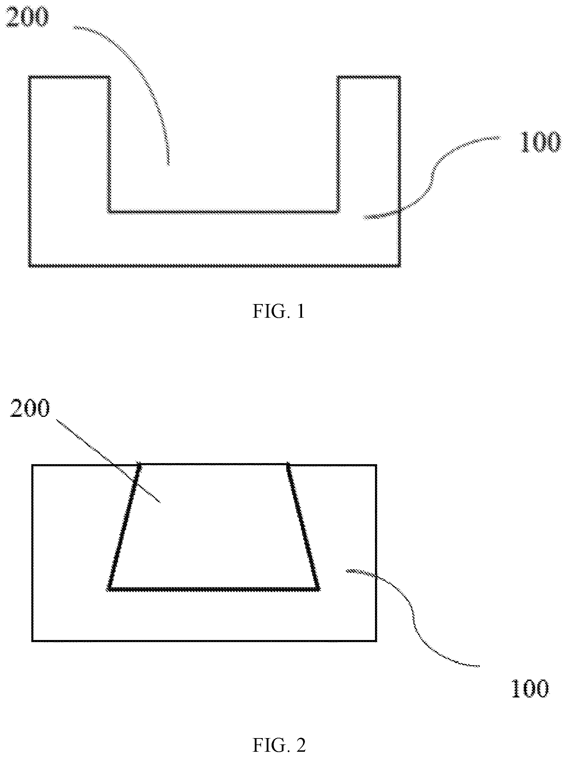

As shown in FIG. 1 to FIG. 3, according to an embodiment of the present disclosure, a biological culture chip is provided. The biological culture chip includes: a matrix 100 and microwells 200. The matrix 100 is formed by a matrix material. An equilibrium swelling ratio of the matrix material of the matrix is approximately 1.25-1.75. The microwell 200 is formed on a surface of the matrix 100, and the microwell 200 has an opening on the surface of the matrix 100. The microwell 200 defines a biological culture space for culturing a biocompatible material.

In the related art, the interface compatibility between a chip matrix and a cell culture medium is poor. The incompatibility between a hard matrix interface and a cell culture medium leads to the inhomogeneity of a 3D culture microenvironment of arrayed cells, and the interfaces of the matrix and the medium intend to slide due to a liquid layer between the two interfaces, which causes damages to the cell array, affecting the realization of cell biology functions and affecting the sufficient capture of cell detection signals and the sensitivity of detection, such as the detection of secretory proteins.

According to this embodiment of the present disclosure, as the matrix material has a certain equilibrium swelling ratio, the entire surface of a biocompatible material, for example, a single cell, cultured by using the biological culture chip can be surrounded by a liquid or gel extracellular matrix material, to provide a uniform microenvironment for culture of the biocompatible material. Therefore, during cell culture, the biological activity of cells can further improved, to promote the realization of cell biology functions.

According to this embodiment of the present disclosure, further, the matrix used in the present disclosure, during the culture of the biocompatible material, can deform according to the growth of cells. In other words, compared with conventional hard matrixes such as glass, silicon wafers, and plastics, the matrix material used in the present disclosure is a soft matrix, so that the force between the matrix and the cells and the force between the matrix and the extracellular matrix material can be reduced, thereby further improving the interface compatibility between the cells and the matrix and the interface compatibility between the matrix and the extracellular matrix material. In addition, according to this embodiment of the present disclosure, in the process of culture, during cell growth, a concave is formed on a contact area between the matrix and a cell. A person skilled in the art can understand that the concave is formed by growth of the cell against the surface of the matrix to cause deformation of the matrix. A person skilled in the art can observe the inner surface of the matrix through a microscope to determine whether there is a concave. According to an embodiment of the present disclosure, a depth of the concave is not less than 5% of a diameter of the cell, such as 10% or 20%. The term "depth of concave" used herein may be determined by comparing an interface micrograph of the substrate with no concave formed to an interface micrograph of the substrate with concave formed. The value of depth herein is defined as a diameter of a sphere of maximum volume that can fit in the formed concave.

Therefore, according to this embodiment of the present disclosure, the soft matrix within microwells with better biocompatibility, cell culture medium compatibility, and mechanical tension can improve the efficiently of capturing and culturing of cells (including single cells and multiple aggregated cells in controlled quantities). According to this embodiment of the present disclosure, the soft matrix is easy to be compatible and fused with different extracellular matrix environments to provide a uniform and integrated 3D culture space for cells, so as to create a microenvironment conducive to survival, proliferation, migration, protein expression and secretion, and stem cell differentiation, and conducive to detection of cell biology functions for 2D-arrayed cells. In addition, considering that the matrix is a soft matrix, it is also easy to isolate single cells from the culture space, without overcoming the adsorption force between a conventional matrix material and the cells.

According to this embodiment of the present disclosure, the matrix material has a solid-liquid phase transition temperature of 40-45°C. According to this embodiment of the present disclosure, the matrix material is capable of being liquid at 37°C. It is to be noted that, the above values of temperature are determined under 1 atmosphere. Therefore, the matrix material can be easily represented as liquid at a temperature appropriately higher than room temperature, and further solidified into a predetermined shape by cooling down. As the matrix material has the above characteristics, when the matrix material is in a liquid state, cytokines can be conveniently added without being inactivated. During the addition of a liquid medium for cell culture, the biological culture chip can absorb the medium to further release these cytokines into the culture space. Based on this, according to this embodiment of the present disclosure, the matrix further includes a cytokine corresponding to a biocompatible material. According to this embodiment of the present disclosure, the matrix is further selected from the group consisting of fetal bovine serum (FBS), protein A/G, collagen, gelatin, or bovine serum albumin (BSA), and a mixture of at least two of them in different proportions.

It is to be noted that, according to this embodiment of the present disclosure, the specific type of the matrix material that can be used is not particularly limited as long as the above performance requirements can be met. Considering better biocompatibility and performance regulation, the matrix material is selected from the group consisting of collagen hydrogel, methylcellulose, agarose gel, and polyacrylamide gel, and a mixture of at least two of them in different proportions. A person skilled in the art can understand that the concentrations of these materials can be adjusted to obtain desired properties of the soft matrix, such as a solid-liquid phase transition temperature.

In addition, according to this embodiment of the present disclosure, the structure and size of the microwell are not particularly limited as long as a space for culture or growth and proliferation of single cells can be provided. According to this embodiment of the present disclosure, the microwell may have a structure such as a cuboid, a cube, or a cylinder. In addition, referring to FIG. 2, according to this embodiment of the present disclosure, a diameter of an opening of a microwell 200 is smaller than a diameter of a bottom portion of the microwell 200. Therefore, single cells can be easily captured for culture without cell escape during the culture. More particularly, according to this embodiment of the present disclosure, a diameter of an opening of a microwell is at most 80% of a diameter of a bottom portion of the microwell, preferably 50%. The term "diameter" used herein is defined as a diameter of a circle of maximum area that can fit in an opening or a bottom portion. The term "depth" of the microwell used herein refers to the shortest distance between a cross section of the opening of the microwell and a cross section of the bottom portion of the microwell. According to this embodiment of the present disclosure, the microwell 200 has an opening diameter of 8-25 µm and a depth of 15-35 µm. In addition, it is found that different opening diameters may be set for different cells. For example, according to this embodiment of the present disclosure, a biocompatible material is B cells, and a diameter of an opening is 8-12 µm; or a biocompatible material is hybridoma cells or tumor cells, and a diameter of an opening is 15-25 µm.

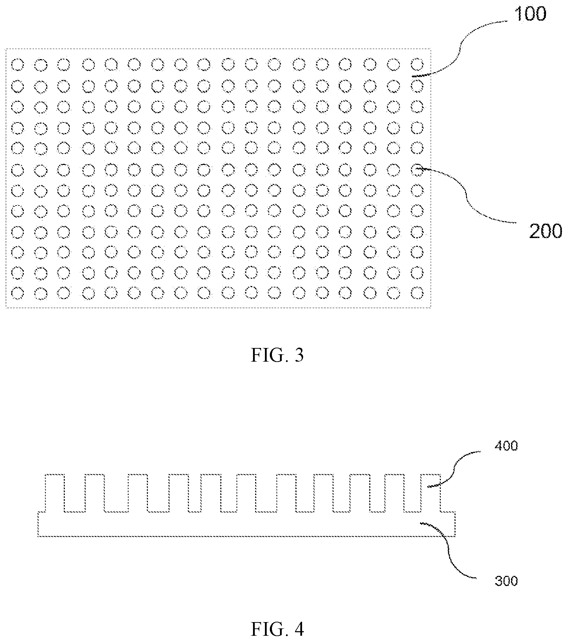

In addition, according to this embodiment of the present disclosure, the biological culture chip may include a plurality of microwells, the microwells form a predetermined pattern, and a distance between two adjacent microwells is 10-100 µm. Therefore, a plurality of single cells can be cultured in batches. According to this embodiment of the present disclosure, when a distance between microwells is 10-100 µm, cells cultured in the microwells do not interfere with each other. Therefore, the uniformity of culture in batches can be improved, and the accuracy and efficiency of detection of cell biology functions can be increased, which facilitates high-throughput collection and analysis of cell signal data. According to this embodiment of the present disclosure, referring to FIG. 3, the microwells on the biological culture chip may form an array. For example, there is an array of tens of thousands or even hundreds of thousands or more of microwells on a chip.

According to this embodiment of the present disclosure, the biological culture chip may be further provided with a positioning marker formed on the matrix. Therefore, a plurality of microwells can be positioned through these positioning markers. The form of the positioning marker is not particularly limited. According to this embodiment of the present disclosure, the positioning marker of a fluorescent material may be provided to perform automatic and precise positioning under a microscope, or a coordinate line may be provided to position every microwells. When positive cells are observed, each positive microwell can be quickly positioned and recorded, which further facilitates subsequent isolation of the positive cells or other components in the microwell.

In addition, according to this embodiment of the present disclosure, a reagent or a biological factor that is helpful for culturing a biocompatible material may be further configured on an inner surface of the microwell. According to this embodiment of the present disclosure, the biocompatible material is hybridoma cells, and a cultural factor of the hybridoma cells is configured on an inner surface of the microwell; the biocompatible material is MCF10A cells, and insulin is configured on the inner surface of the microwell; or the biocompatible material is B cells, and at least one of CD40L, IL2, or IL10 is configured on the inner surface of the microwell or a mixture of at least two of them in different proportions.

According to this embodiment of the present disclosure, a biological culture chip (also referred to as cell array chip) with a soft matrix such as a solidified hydrogel is provided can resolve a series of problems in 2D array and 3D culture of cells *in vitro,* including: cell capture, formation of a flux-controlled cell array, cytocompatibility of a chip material, compatibility of a chip material and a cell medium, realization and accurate detection of arrayed-cell biology functions, and the like. Arrays with microwells of different diameters and depths can be provided on a chip with a gel soft matrix to satisfy different needs. The mechanical strength of the microwell can be determined by the concentration of the gel soft matrix. The mechanical strength is adjusted according to different cell types and different purposes of detection of biology functions to obtain an optimal mechanical strength of the microwell. In addition, the diameter and depth of the microwell is controllable to facilitate aggregation of single-cell array of different cells or multiple cells in controlled quantities in the microwell. The distance between microwells is controllable to determine the density of the cell array on the chip and the flux of the cell array on the chip per unit area. The gel soft matrix can provide similar characteristics to the extracellular matrix. It has good cytocompatibility, provides cells with water, nutrition, and soft space, thereby avoiding an improper mechanical strength that cannot be adjusted according to different cells cultured by a chip with a hard matrix, and avoiding chemical molecule residues from chemical modification usually required for the chip with a hard matrix. The gel soft matrix has excellent compatibility with a cell medium or a 3D-cultured hydrogel biocompatible material, which ensures a uniform microenvironment around arrayed cells, and ensures the integrity and accuracy of detection of cell functions.

### Preparation of Biological Culture Chip

According to another aspect of the present disclosure, a template for preparing the above biological culture chip is provided. Referring to FIG. 4, according to an embodiment of the present disclosure, the template includes: a base plate 300; and a microcolumn 400, configured on a surface of the base plate 300, wherein the microcolumn 400 is adapted for the microwell 200 in a form-adapting manner. According to this embodiment of the present disclosure, the matrix material is solidified on the template, so that the above biological culture chip can be effectively prepared.

According to this embodiment of the present disclosure, the microcolumn has a hydrophobic surface. Therefore, the formed biological culture chip can be effectively peeled off from the template. In addition, according to this embodiment of the present disclosure, a surface of the microcolumn is suitable for binding to a biological factor through a hydrophobic effect. Subsequently, by applying the matrix material, the biological factor can be transferred onto the matrix material as the biological factor binding to the inner surface of the microwell. As described above, different biological factors may be provided according to different culture objects and purposes.

According to another aspect of the present disclosure, a method for preparing the above biological culture chip is provided, including: (1) applying a liquid biocompatible material to the template above as a matrix material, and solidifying the matrix material; and (2) separating the solidified biocompatible material from the template to obtain the biological culture chip. By this method, the above biological culture chip can be effectively prepared. According to this embodiment of the present disclosure, a biological factor or a reagent is pre-adsorbed on a surface of the template.

More particularly, according to this embodiment of the present disclosure, a method for preparing the above biological culture chip includes the following steps.

Step 1. Modify a template. Cytokines and other substances required in a biological culture microenvironment are allowed to bind to the template through a hydrophobic effect. More particularly, a material used for modification is formulated into a modification solution (with a solvent of PBS) in a concentration of 1 µg/mL; the template is soaked in the modification solution to react at room temperature for 4 h; and after taken out, the template is rinsed with fresh PBS and deionized water in succession for 3 times, blow-dried with nitrogen gas, and preserved in a dry place for use.

Step 2. A melt matrix material, for example, a gel matrix, is placed on the template with a column array to cover it, after the matrix material, for example, a gel matrix, is cooled down and solidified, the template is removed, to form a soft matrix-based chip with a microwell array, having a certain thickness and hardness. More particularly, an agarose gel liquid with a concentration of 0.6%-1.2% may be poured on the template and left at room temperature for 5 min; and after the liquid is solidified, the template is removed, to form a biological culture chip. Each microwell in the chip contains a factor or treatment drug required for the microenvironment.

### Method for Culture Using Biological Culture Chip

According to another aspect of the present disclosure, a method for culturing cells using the above biological culture chip is provided, comprising: arranging cells within the microwell; and exposing the biological culture chip to an environment with a predetermined condition. By using this method, a uniform extracellular matrix-like microenvironment can be provided for cultured cells, thereby effectively maintaining the viability of the cells and improving the efficiency of cell culture. According to this embodiment of the present disclosure, at most one cell is arranged within each microwell.

According to this embodiment of the present disclosure, the soft matrix of the chip and a cell medium have good compatibility, which helps provide a uniform and proper microenvironment for 3D culture of high-throughput arrayed cells, helps realize arrayed cell biology functions, and helps sufficient capture and accurate analysis of cell biochemical reaction signals (such as secretory protein signals).

In addition, according to this embodiment of the present disclosure, the chip with a soft matrix can better produce an extracellular matrix-like interfacial effect between cells and the chip, so that the 3D-cultured cells grow in an environment closer to the living environment *in vivo,* which is conducive to the survival of cells and the realization of biological functions.

According to this embodiment of the present disclosure, a cell array of the chip with a soft matrix is formed relying on cell gravity and a biocompatible material such as an extracellular matrix added into the microwell to attract and stabilize the cells, instead of chemical modification of the chip.

According to this embodiment of the present disclosure, the chip with a soft matrix is conducive to picking up positive cells (single cells or multicellular spheroids) under a microscope using a glass needle. However, it is difficult to picking up cells from a chip with a hard matrix under a microscope using a glass needle, because its hard matrix interface is prone to cause damage to the glass needle or the cells.

### Example 1 Preparation of Chip

### 1.1 Preparation of a chip with a soft matrix

The template with a column array shown in FIG. 4 was selected and classified, according to different cell types, into the following two types:
A. a template suitable for generalized cells (generally with a diameter of 15-20 µm), with microcolumns of a height of 35 µm and a cross-sectional diameter of 20 µm, where a distance between adjacent microcolumns is 30 µm; and
B. a template suitable for small cells (generally with a diameter of 8-10 µm), with microcolumns of a height of 20 µm and a cross-sectional diameter of 10 µm, where a distance between adjacent microcolumns is 30 µm.

A modification solution containing methylcellulose or low melting point agarose was prepared with PBS as a solvent.

The template was soaked in the modification solution at room temperature for 4 h and then taken out, and the template was rinsed with fresh PBS and deionized water in succession for 3 times, blow-dried with nitrogen gas, and preserved in a dry place for use. Therefore, the template used for preparing a chip is modified, so that cytokines and other substances required in a microenvironment bind to the template through a hydrophobic effect.

A melt hydrogel matrix was placed on the modified template (with the column array) to cover it. After the hydrogel matrix was cooled down and solidified, the template was removed, and a soft matrix-based chip with a microwell array was formed, which has a thickness of about 300-500 µm. More particularly, an agarose gel liquid (with a swelling ratio of about 1.25-1.75) with a concentration of 0.6%-1.2% was poured on the template and left at room temperature for 5 min; and after the liquid was solidified, the template was removed, and a biological culture chip was formed. Each microwell in the chip contained a factor or treatment drug required for the microenvironment.

The following table shows the composition of a chip with a soft matrix:

| Number of chip with a soft matrix | Composition of the soft matrix | Composition of modification solution | Factor or drug contained in matrix |
|---|---|---|---|
| Chip 1 with a soft matrix | 1% of agarose gel | 0.1% of methylcellulose | B12, IL6, IL1 |
| Chip 2 with a soft matrix | 0.8% of agarose gel | 0.2% of low melting point agarose | Human insulin |
| Chip 3 with a soft matrix | 1.2% of agarose gel | HFF-derived ECM | Polypeptide coupled to antigen or carrier protein |

### 1.2 Preparation of a chip with a hard matrix

A chip 1 with a hard matrix and a chip 2 with a hard matrix were prepared using hard matrix materials glass and silicon wafer respectively by the method described in CN108102913A "3D Cell Culture Chip Based On Soft Lithography, Preparation Method and Application Thereof", and details are not described herein.

Example 2 Comparison of Chip with Soft Matrix and Chip with Hard Matrix for Cell Culture

### 2.1 Comparison of efficiency of array formation

According to the following steps, mouse hybridoma cells, human tumor cells (MCF7), human umbilical cord mesenchymal stem cells, and mouse B cells were cultured by using the chips 1, 2, and 3 with a soft matrix, and the above cells were also cultured by using the chip 1 with a hard matrix (glass substrate) in parallel. These cells were cultured by a conventional cell culture method (specifically referring to *Cellular and Molecular Biology Experiment Guide)* for 15 min, to determine the array efficiency. The determination of the array efficiency was as follows.

The quantity of single cells per unit area (1 mm×1 mm) was observed and counted, and divided by the total quantity of chambers, to obtain an efficiency of obtaining a single-cell array.

In order to ensure that only one cell can be accommodated in each chamber, the specification of the chip was controlled to limit the space of the chamber. In addition, a very small amount of doubles produced due to uncontrollable factors were easily discriminated using a microscope and excluded from the statistical range.

The results of the above determination are as follows.

| | Mouse hybridoma cell | Human tumor cell (MCF7) | Human umbilical cord mesenchymal stem cell (MSC) | Mouse B cell |
|---|---|---|---|---|
| Chip with a hard matrix* | 56.32% | 32.93% | 30.43% | 49.03% |
| | 42.17% | 30.22% | 35.88% | 41.28% |
| | 48.99% | 34.87% | 32.03% | 45.39% |
| Mean | 49.16% | 32.67% | 32.78% | 45.23% |
| Chip with a soft matrix* | 97.25% | 92.97% | 90.33% | 93.29% |
| | 96.93% | 93.46% | 92.39% | 94.24% |
| | 97.82% | 95.21% | 91.57% | 93.33% |
| Mean | 97.33% | 93.88% | 91.43% | 93.62% |

| | | | | |
|---|---|---|---|---|
| ^{∗}Single cells culture in the chips (20 µm/30 µm, 10 µm/30 µm). The first number represents a diameter of the chamber, and the second number represents a distance between adjacent chambers. In the above table, except for mouse B cells which were cultured by using the chip with a specification of 10 µm/30 µm, the other types of cells were cultured by using the chip with a specification of 20 µm/30 µm. | | | | |

It can be seen from the above data that the array efficiency of the chip with a hard matrix is about 32-50%, indicating that the chip with a hard matrix is not conducive to the formation of a cell array. The array efficiency of the chip with a soft matrix is very high, about 91-98%, indicating that the chip with a soft matrix is very effective for the formation of a cell array.

The inventor believes that the above difference mainly results from the hard matrix of the chip, such as glass or silicon wafer. As the material of the hard matrix does not have voids and water permeability, gas in the small chamber can barely be discharged completely. As a result, the subsequently inoculated cells cannot occupy a proper position through gravity sedimentation. However, the soft matrix of the chip, such as a hydrogel or other biocompatible materials, has a certain pore size and water permeability, so when cells are inoculated, the culture medium can penetrate from surrounding of the chip to completely discharge gas from the small chamber, which is conducive to cell sedimentation to form an array. Based on the above, the chip with a hard matrix and the chip with a soft matrix that have the same quantity of chambers have a large difference in the proportions of chambers that are used for forming the cell array effectively. Therefore, the efficiency of the chip with a hard matrix for cell screening is much lower than that of the chip with a soft matrix.

### 2.2 Comparison of cell viability on the chip

By the method of 2.1, the cells were cultured by using the chip with a hard matrix and the chip with a soft matrix respectively, and cell viability was detected according to the following method. The cell viability on the chip was identified by the live/dead assay (Thermo, L34951). Dead cells exhibit red fluorescence, and live cells exhibit green fluorescence.

The results are as follows:

| Chip type | Average cell viability |
|---|---|
| Chip with a hard matrix | 56.4% |
| Chip with a soft matrix | 98.7% |

It can be learned from the above data that the chip with a hard matrix is not conducive to cell culture and survival, and easily leads to cell apoptosis and necrosis, which is not helpful in cell proliferation. The main reason for this is that the hard matrix material does not have physical elasticity and cannot provide a uniform extracellular matrix-like microenvironment (with the characteristics similar to the living space *in vivo*) required for 3D culture of cells in all directions, which changes the pathways of cell survival, proliferation, senescence, and apoptosis, leading to cell senescence, apoptosis, or necrosis. However, the soft matrix material has better plasticity and has sufficient water and supporting space, which can be adjusted with the change of requirements for space of cell survival and can be compatible and fused with the ECM medium for 3D cell culture to provide a uniform and proper microenvironment for cells, thereby helping the survival of cells and the realization of biological functions (such as factor and antibody secretion). In addition, during the preparation of the chip with a soft matrix, various factors or materials required for culture of different cells can be appropriately added to construct a microenvironment for cell culture and survival, which greatly improves cell survival and proliferation. However, it is difficult for the hard matrix material to achieve this. Various factors or materials are usually added to a culture medium or chemically modified to the surface of the hard matrix material, so it is difficult to sufficiently disperse the factors or materials around the cells, or the residual chemical substances will be toxic to the cells.

### 2.2 Comparison of capacity of picking up single cells on the chip

A single-cell chip is generally used to obtain and analyze single-cell information with special significance. Therefore, target single cells need to be isolated for subsequent operations. Based on this, the inventor tried picking up single cells on the chip with a soft matrix and the chip with a hard matrix respectively using a capillary needle.

The comparison results are as follows:

| Chip type | Success rate of picking (single cells) | Efficiency of needle reuse |
|---|---|---|
| Chip with a hard matrix | 23.5% | Low |
| Chip with a soft matrix | 95.6% | High |

The inventor believes that the reason for the low success rate of picking up single cells in the chip with a hard matrix is mainly because all the cells are suspension-cultured without any restraint, so when the target cells are picked up using a needle, non-target cells near the target cells are likely to be picked up; moreover, when the needle moves close to the target cells, the target cells are likely to be affected by a slight force (the force generated due to movement of the needle in the liquid) to separate from the chip or be pushed away, resulting in a failure in picking. However, the material of the soft matrix of the chip is a hydrogel, which easily produces a proper adhesion effect with the cells, so that the cells are not completely suspension-cultured, thereby resolving the problems caused by the hard matrix and increasing the success rate of picking.

In addition, when there is contamination, deformation, or other problems, the needle, as a consumable, can be replaced at any time during cell picking, so as to increase efficiency. However, needle breakage or other problems caused by improper operation will greatly affect the picking efficiency. This is because the replacement of the needle and the precise adjustment of the position take time. The hard matrix does not have elasticity. During the operation under a microscope, it is necessary to keep a very small distance between the needle and the chip, or even touch the surface of the chip, to ensure a weak suction force can be released to the target cells as much as possible for successful picking. However, manual or semi-automatic operations are difficult to complete accurately every time, so it is common for the needle to deform or break when touching the chip. Because of good physical elasticity, the soft matrix has a high tolerance to the excessive contact of the needle, which allows an operator to detect and make adjustments in time.

### Example 3: Screening for Monoclonal Antibody

In this example, a chip with a soft matrix was prepared by the method in Example 1 for screening for a single cell-derived monoclonal antibody against a novel coronavirus S1 protein.

### 3.1 Immunization for mice

5-week-old BALB/c female mice were used, purchased from Shanghai Silaike Experiment Animal Co., Ltd. The mice were laboratory-bred in an animal room for one week to adapt to the environment. Then, the 6-week-old mice were prepared for immunization. The novel coronavirus S1 protein, purchased from Novoprotein Technology Co., Ltd., was dissolved in PBS, and the concentration was adjusted to 1 mg/mL before immunization. Before injection, the antigen was emulsified through mixing using a syringe. The specific immunization schedule and conditions are shown as follows.

| Number of immunization operations | Time | Antigen dosage | Adjuvant selection and dosage | Immunization manner |
|---|---|---|---|---|
| First immunization operation | Day 0 | 100 µg | Complete adjuvant, 100 µL | Intraperitoneal |
| Second immunization operation | Day 14 | 50 µg | Incomplete adjuvant, 100 µL | Intraperitoneal |
| Third immunization operation | Day 28 | 50 µg | Incomplete adjuvant, 100 µL | Intraperitoneal |
| Booster immunization operation | Day 35 | 100 µg | No adjuvant | Intraperitoneal |

After the second and third immunization operations, the mice were blood-sampled from orbits to measure an antibody titer in the serum. The antigen dosage for the third or booster immunization operation was adjusted correspondingly according to the antibody titer, until the immune response of the mice to the antigen reached an optimal state.

### 3.2 Cell fusion

According to the results of titer in the serum 3 days after the booster immunization operation, a mouse with the highest titer was selected for subsequent fusion. Before the fusion, all the blood of the mouse was collected and the serum was obtained for use.

SP2/0 cells were prepared in advance (3-5 days, thawed in advance). 50 mL of blank 1640 medium and 500 mL of sterile water were preheated in an incubator in advance overnight for the fusion on the next day. PEG used in the fusion was preheated in an incubator before the removal of spleen.

The immunized mouse was cervically dislocated and soaked in 75% ethanol for 3-5 min for sterilization. The removal of spleen was carried out in a biosafety cabinet. The sterilized mouse was taken out and left to drain off the ethanol, and then placed on paper towels with its abdomen facing upward. A small slit (1 cm) was cut on the upper left abdomen using a pair of scissors, and the spleen (posterior and inferior to the left lobe of the liver) was removed using a pair of tweezers. During the removal, be careful not to puncture the spleen and remove excess connective tissue.

The removed spleen was placed in a 40 µm sieve, and then the sieve was placed in a 50 mL centrifuge tube filled with the blank 1640 medium. Be careful not to add too much liquid to prevent the medium from overflowing during grinding of the spleen. During the grinding, the spleen needs to be fully exposed to the medium. To facilitate isolation of splenocytes through grinding, small holes were pierced in the spleen using a syringe needle before the grinding, or a few small slits were cut on the spleen using a pair of scissors. The spleen was ground to pass the spleen contents fully through the sieve and leave the remaining connective tissue and the like. The ground spleen was centrifuged at room temperature at 1300 rpm for 8 min twice to collect splenocytes. After a supernatant was removed, 10 mL of PBS was added to resuspend the splenocytes. 30 mL of red blood cell lysis buffer was added to lyse at 4°C for 15 min and uniformly mixed twice upon the lysis. The resulting mixture was centrifuged at room temperature at 1300 rpm for 8 min to collect splenocytes. The splenocytes were resuspended in PBS and centrifuged twice until the lysis buffer was completely removed.

The splenocytes and tumor cells were counted separately. A ratio of splenocytes to tumor cells was adjusted to 5:1. The splenocytes and tumor cells were mixed in a 50 mL centrifuge tube with the above ratio, and centrifuged at room temperature at 1300 rpm for 8 min to collect mixed cells. A supernatant was removed completely (as the residual supernatant will affect fusion efficiency). The bottom of the centrifuge tube was patted with the hand, or the centrifuge tube was moved back and forth on a ventilation mesh plate of the biosafety cabinet, to disperse precipitated cells and finally obtain a cell homogenate. Cell fusion was carried out with the PEG preheated to 37°C in a 37°C water bath, and terminated with the blank 1640 medium. The specific steps of fusion were as follows:
PEG was added dropwise along the tube wall using a pipette, with the outlet tip of the pipette as close to the cells as possible, by 1 mL within 1 minute. The centrifuge tube was rotated and slightly shaken while the PEG was added dropwise, to ensure that the cell homogenate can be fully and evenly exposed to PEG.

The cell homogenate was left to stand at room temperature for 1 minute.

The preheated blank 1640 medium was added dropwise by 1 mL within 1 minute. The centrifuge tube was rotated and slightly shaken while the medium was added dropwise, to ensure that the cells can be fully and evenly exposed to the medium.

The preheated blank 1640 medium was added dropwise by 2 mL within 1 minute. The centrifuge tube was rotated and slightly shaken while the medium was added dropwise, to ensure that the cells can be fully and evenly exposed to the medium.

The preheated blank 1640 medium was added dropwise by 5 mL within 1 minute. The centrifuge tube was rotated and slightly shaken while the medium was added dropwise, to ensure that the cells can be fully and evenly exposed to the medium.

The preheated blank 1640 medium was added dropwise by about 21 mL. The centrifuge tube was rotated and slightly shaken while the medium was added dropwise, to ensure that the cells can be fully and evenly exposed to the medium. After the fusion was completed, the resulting mixture was centrifuged at room temperature at 1300 rpm for 8 min to collect the fused cells.

The fused cells were resuspended (with purging gently) with a complete medium (20% FBS, 1% P/S, 1×HAT, 1×hybridoma growth factor (HGF)) added in an amount of 1×10⁶ splenocytes/mL. The resuspended cells were inoculated into a culture dish to be cultured for 3-5 days, during which the medium was changed once.

### 3.3 Preparation of Chip

A template was prepared and modified with a layer of HGF (using an HGF-containing PBS solution as a modification solution which is beneficial to the survival and proliferation of hybridoma cells). About 500 µL of gel for preparing the chip was drawn (by aseptic technique) and added dropwise to the center of the template (sometimes called the stamp) and left to stand at room temperature for 10 minutes (to effectively prevent excessive drying). After the gel solidified, the template and the chip were gently separated from each other, during which displacement needed to be avoided, otherwise the integrity of the structure will be destroyed. The prepared chip was preserved in a culture dish with a diameter of 10 cm for use.

### 4.4 Formation of cell array

The fused cells obtained in 4.3 were collected and washed with PBS or the medium for 1-2 times to remove excess culture components (antibodies in the culture supernatant will cause high background of detection). It is recommended to adjust the concentration of the cells to 8×10⁵-1×10⁶/mL. The cell suspension was added dropwise to the surface of the chip in about 500 µL/chip. The chip was placed in an incubator to stand for 10 min. Excess cell suspension was recovered using a pipette, then about 300-500 µL of culture medium was drawn and slowly added dropwise to the chip tilted by about 30-45° from above to rinse excess cells (with no medium flowing out of the chip), and then excess medium was removed by using a vacuum pump. The formation of the cell array was observed under a microscope to determine whether to proceed with subsequent operations.

### 4.5 Antigen labeling and screening for positive hybridoma cells

Thermo's protein labeling kit (A30006) was used with basic operation steps according to the instructions.

In short, within 10 minutes of waiting for the formation of the cell array, a freezing container was taken out, and labeled antigens and a self-made 3D culture hydrogel were arranged in the freezing container for use. 50 µL of complete medium was added into a 1.5 mL sterile centrifuge tube, and placed in the freezing container for use. After the cell array was formed, 50 µL of hydrogel was added into each centrifuge tube and mixed uniformly. 70-80 µL of well-mixed hydrogel was drawn out and added dropwise from one corner of the chip, while the chip was gently shaken to allow the hydrogel to fully cover the structure. The chip was then placed in an incubator to stand for 5 minutes, during which the chip was kept level. The chip was moved into a culture dish with a diameter of 3.5 cm by using a syringe needle and tweezers and placed in an incubator, and then a screening solution was added (in a volume of 2 mL in a ratio of fluorescent-labeled antigen: complete medium = 1:(200-1000)). The chip was left to stand for at least 3 hours.

### 4.6 Single-cell picking

After screening for 3-5 hours, the condition of signals was first observed under a fluorescence microscope, until positive signals appear and have the proportion that satisfies the requirement, then the cells were ready for picking.

### 4.7 Monoclonal culture and identification

The cells with the positive signals were picked up and released into a 96-well plate, and then subjected to microscopic examination within 1-2 hours to determine whether there is a single cell in each well. After culture for 7-14 days, a supernatant of stably proliferating cells in each well was tested by ELISA to determine whether the cells secrete antigen-specific antibodies, that is, whether the cells are positive clones. The positive clones were continuously cultured to proliferate, and the secretion of the antibodies was identified again before cryopreservation.

### 4.8 Screening results

Through screening, 15 cell lines that can secrete specific antibodies were obtained, including 14 monoclonal cell lines. All the obtained clones were derived from different mice or different hybridoma cells of the same mouse, which can sufficiently ensure the diversity of clones.

Compared with the conventional method (limiting dilution), the method of the present disclosure has very significant advantages, specifically as follows:

| Screening method | Time | Quantity of clones | Quantity of 96-well plates | Quantity of personnel |
|---|---|---|---|---|
| Limiting dilution | 3-4 months | 2-5 | Hundreds | 3 or more |
| Method of this application | 1.5-2 months | At least 15-50 | 1-2 | 1 |

Therefore, compared with conventional limiting dilution, the method of the present disclosure has an absolute advantage in terms of screening efficiency and screening cost, resulting in a decrease of about 90% in the overall cost and an increase of at least 10 times in the screening efficiency. This is only the result obtained without conducting a comprehensive screening, and personnel and time can be arranged more properly according to the urgency degree of a project. Based on this, it is believed that the screening efficiency can be effectively increased by 30 times or more.

### Example 5: Screening for Paired Antibody

### 5.1 Acquisition of labeled antibody and fluorescent-labeling

A monoclonal cell line with a high antibody titer was selected according to the screening results in Example 4. Ascites was developed by following steps.
① 2 female B/C mice of 8-10 weeks of age were prepared for each group. The Freund's incomplete adjuvant was intraperitoneally injected into the mice in 500 µL/mouse for sensitization.
   5×10⁵ cells were inoculated intraperitoneally. Note: fewer cells injected cause slow formation of ascites, but lead to a higher titer; the hybridoma cells to be injected need to be detected for the antibody titer; before inoculation, the cells need to be washed for 2-3 times with a serum-free medium in about 0.2-1 mL for each mouse.
(2) The ascites and survival of the mice were closely observed on second day after inoculation. About 7 days later, on a condition of the mice are active and have obvious shifting dullness in the abdomen, the ascites fluid may be drained 2 times; once the mice are found inactive, they have to be sacrificed and their ascites fluids have to be drained.

Draining ascites fluid from a living body: the position where a syringe needle was to be inserted (approximately on the right side of the midline of the abdomen) was wiped with an alcohol-soaked cotton ball, the syringe with remaining air was inserted and then the plunger was slowly pulled out, so that the pressure generated in the abdomen makes the ascites fluid flow out slowly. The syringe may be lifted up slightly during needle insertion until feeling no obstruction.

Draining ascites fluid after being sacrificed: a small slit was gently cut on the outer skin of the abdomen using a pair of scissors and tweezers, two sides of the outer skin were gently pulled apart using two pairs of hemostatic forceps to expose the abdominal cavity, the inner skin was cut using another pair of scissors and another pair of tweezers within a small area, so that the ascites fluid can be drained using a pipette or a straight glass tube.

In normal cases, 2-3 mL of ascites fluid can be collected from each mouse one time. In response to a mouse is in good condition, its ascites fluid can be collected on alternate days. The concentration of the antibody was 0.5-5 mg/mL.

③ The ascites fluid was collected in a 15 mL centrifuge tube and centrifuged at 800 rpm for 30 min (or at 2000 r/min for 5 min) to remove cells and fat. The freshly collected ascites fluid contains a large amount of impurities such as fat, macrophages, hybridoma cells, epithelial cells in the abdominal cavity, and cellular components of blood, especially the cellular components. On a condition of they were not removed, the cellular components existing in the ascites fluid for a long time may slowly rupture and release protein into the ascites fluid, which increases impurities in the ascites fluid, making purification difficult. Therefore, the collected ascites fluid, whether for long-term preservation or immediate purification, should be centrifuged first to remove cellular components and fat (fat has a low density and generally floats on the surface of the ascites fluid, so it can be drawn out using a pipette).

The purification of the antibody and the preparation of the fluorescent-labeled antibody were carried out in specific operation steps according to the instruction in the Thermo's kit (89953 A30006).

### 5.2: Screening for paired antibody

The specific steps were the same as those described in Example 4, but the fluorescent-labeled antigen was replaced with an antigen-fluorescent-labeled first antibody complex in a ratio of 2:1.

In this step, another method as follows with specific steps the same as those described in Example 4 can also achieve the same screening purpose and effect. A 3D medium containing the antigen was added to the chip containing 2D-arrayed hybridoma cells for culture for 2 h to fully bind the antigen to the antibody secreted by the hybridoma cells, then the 3D liquid medium was removed, and a 3D medium containing the fluorescent-labeled first antibody was added for culture for 2-3 h.

### 5.3 Screening results

Two high-titer clones of the antibody against the novel coronavirus S1 protein obtained in Example 4 were selected as first antibodies, namely S1-6S and RBD-10S respectively. After the above screening, the results are as follows:

| First antibody | Paired antibody obtained through screening |
|---|---|
| S1-6S | 6S-1S, 6S-7S, 6S-8S, 6S-10S |
| RBD-10S | 10S-2S, 10S-5S, 10S-8S |

There were 4 antibody pairs against the S1 protein, and there were 3 antibody pairs against the RBD protein. The paired antibodies were derived from different mice or different hybridoma cells of the same mouse, which can ensure the diversity of antibody pairs.

### Example 6: Screening for single cells that secrete a specific factor by using a chip with a soft matrix

Referring to Examples 4 and 5, CHO cells that stably express VEGFA were obtained through screening. More particularly, the chip preparation, 2D array of single cells, antigen-antibody reaction of single cells, identification and picking of positive single cells, and the like were the same as those described in Example 4 or 5. The difference lies in that in this example, the fluorescent-labeled antigen for immunofluorescence was replaced with a fluorescent-labeled specific antibody for the secretion of VEGF. The screening results are as follows:

| Labeled antibody | Quantity of cells picked up after screening | Quantity of cell lines that are capable of stable expression |
|---|---|---|
| Anti-VEGFA (Abeam, abl316) | 58 | 52 (89.6%) |
| RBD-10S (Proteintech, 6628-1-1g) | 49 | 42 (85.7%) |

Two VEGFA antibodies derived from different sources were used for testing. After drug selection, 58 and 49 positive cells (that can secrete the factors) were separately picked up. Through subsequent culture and verification, 52 and 42 CHO cell lines that can stably express and secrete VEGFA were obtained respectively with positive rates of 89.6% and 85.7% respectively. Conventional screening and isolation of stably transfected CHO cell lines are still carried out by limiting dilution as described above. Our technique shows great advantages in terms of time, material and personnel consumption.

In addition, referring to Examples 4 and 5, single cells that can secrete other factors were also obtained through screening. Various cells that can express secretory proteins include HEK293, CHO, Hela, MCF10A, HFF, and the like. Factors include various growth factors and cytokines that can be secreted out of cells, such as VEGF, SDF-1a, PEDF, and FGF. Labeled proteins that specifically bind to proteins secreted by cells include antibodies specific to secretory proteins and receptor-binding domains. Details are not described herein.

### Example 7: Sphere Formation of Mouse Neural Stem Cells

Neural stem cells are a cell population existing in the nervous system, which are multipotent to differentiate into neurons, astrocytes, and oligodendrocytes to produce a large number of brain cells, and are self-renewing to provide a large number of cells for brain tissue. Therefore, how to better culture neural stem cells in vitro is the basis and foundation of scientific research.
(1) Extraction of neural stem cells of fetal mice. A C57BL/6 mouse at a gestation period of 14 days was cervically dislocated and soaked in 75% ethanol, and its abdomen was cut and open, and a string of fetal mice (with the umbilical cord cut off) were taken out and placed in a culture dish containing pre-cooled PBS. The placenta was peeled from the fetal mice using tweezers, and the fetal mice were placed in a new culture dish containing pre-cooled PBS (generally, 8-11 fetal mice develop in a pregnant mouse, 6 at least, and 13 at most). Heads of the fetal mice were twisted off and placed in a new culture dish containing PBS (taking 2 fetal mice for each dish). The cerebral cortex was taken out under a stereo microscope, the brain tissue connected to the cerebral cortex, such as the olfactory bulb and meninges should be removed. The cerebral cortex was placed in a 15 mL centrifuge tube, purged with a Pasteur pipette until no visible tissue pieces, and centrifuged at 800 rpm for 2 min. The supernatant was drawn out for use. A new PBS buffer was added in the precipitate and purged into a suspension. The suspension was centrifuged at 800 rpm for 2 min. The supernatant was drawn out and mixed with the supernatant obtained from the first centrifugation, and then centrifuged at 1200 rpm for 2 min. The supernatant was removed. The precipitate was purged with a proliferation medium to obtain an NPC suspension. The NPC suspension was filtered with a 40 µm filter membrane to remove undispersed clumps. The NPC suspension was drawn into a T25 flask for suspension culture. Do not shake the flask for the first two days of culture. Generally, the cells start to passage on the 6th or 7th day depending on the size of the cell spheroids. The cells were collected and centrifuged at 800 rpm for 2 min. The supernatant was removed. A PBS buffer was added to purge the precipitate, and centrifuged at 800 rpm for 2 min to remove the remaining medium. The supernatant was removed. 1-2 mL of Accutase or TryplE cell dissociation solution was added in the tube containing the precipitate to dissociate the cells, and the tube were placed in an incubator at 37°C for 5-10 min, during which the tube may be flicked by fingers. Ca/Mg²⁺-free PBS was added in a 5-fold volume of the above solution to stop the dissociation (or 4-5 mL of preheated medium was added for resuspension), purged multiple times, and centrifuged at 1200 rpm for 2 min. The supernatant was removed. 1 mL of proliferation medium was added (or the proliferation medium may be added more to avoid more loss in subsequent filtration) to purge the precipitate into a suspension. The suspension was filtered with a 40 µm filter membrane to remove the cell spheroids that were not dissociated completely, to obtain an NPC suspension. The cells in the NPC suspension were counted by using a hemocytometer to calculate the concentration of the cells. 10×10⁶ cells were inoculated into a new T25 flask for culture.
(2) Inoculation on a chip. The specific steps were performed with reference to Example 4 or 5. In this example, the specification of the chip is 12 µm/30 µm, the factors added in the soft matrix are EGF and bFGF with final concentrations of 20 ng/mL and 10 ng/mL respectively.
(3) Results and analysis. The method of the present disclosure is compared with the conventional culture method with specific test data shown as follows. From this, it can be seen that a chip with a soft matrix helps increase cell viability and well maintain cell sternness.

### Example 8: Sphere Formation of Tumor Cells (MCF7)

Studies of tumor growth and drug sensitivity have largely relied on an *in-vitro* monolayer tumor model (2D cell culture). This model lacks many hallmarks of disease, such as hypoxia, altered cell-to-cell contacts, and altered metabolism. Through years of research, it is believed that the phenotype of a 3D tumor model is closer to real cancer tissue, which can provide more accurate models and data for drug screening.
(1) MCF7 cell culture and inoculation on a chip. MCF7 cells were cultured in a DMEM medium containing 10% of FBS and 1% of P/S. When the culture area reached 80% of the surface area of the culture dish, the MCF7 cells were dissociated and inoculated on the chip. The specific steps of the inoculation on the chip were carried out with reference to Example 4 or 5.
(2) Continuous culture. The chip containing the cells was placed in an incubator for continuous culture for 3-5 days, during which the specific proliferation of the cells was constantly observed. Through culture, an array of cell spheroids derived from single MCF7 cells was formed on the chip.

This 3D culture of the tumor cells (or the culture of "cell mass") is able to closely replicate many key properties of the original tumor. In addition, the array culture is suitable for large-scale drug screening to detect drug sensitivity associated with genetic changes, establishes an experimental basis for personalized treatment, and can optimize clinical outcomes for cancer patients.

In the description of the present disclosure, it should be understood that orientation or position relationships indicated by the terms such as "center", "longitudinal", "transverse", "length", "width", "thickness", "on", "under", "front", "back", "left", "right", "vertical", "horizontal", "top", "bottom", "inside", "outside", "clockwise", "counterclockwise", "axial direction", "radial direction", and "circumferential direction" are based on orientation or position relationships shown in the accompanying drawings, and are used only for ease and brevity of illustration and description of the present disclosure, rather than indicating or implying that the mentioned apparatus or component needs to have a particular orientation or needs to be constructed and operated in a particular orientation. Therefore, such terms should not be construed as limiting of the present disclosure. In addition, features defined by "first" and "second" may explicitly or implicitly include one or more such features. In the description of the present disclosure, unless otherwise stated, "a plurality of" means two or more. In the description of the present disclosure, that a first feature is "on" or "under" a second feature may include that the first and second features are in direct contact, or may include that the first and second features are not in direct contact but in contact by using other features therebetween.

In the description of the present disclosure, that the first feature is "on", "above", or "over" the second feature includes that the first feature is right above and on the inclined top of the second feature or merely indicates that a level of the first feature is higher than that of the second feature.

In the description of the present disclosure, it should be noted that, unless otherwise explicitly specified or defined, the terms such as "mount", "connect", and "connection" should be understood in a broad sense. For example, the connection may be a fixed connection, a detachable connection, or an integral connection; or the connection may be a mechanical connection or an electrical connection; or the connection may be a direct connection, an indirect connection through an intermediary, or internal communication between two components. A person of ordinary skill in the art may understand specific meanings of the foregoing terms in the present disclosure according to specific situations.

In the descriptions of this specification, descriptions using reference terms "an embodiment", "some embodiments", "an exemplary embodiment", "an example", "a specific example", or "some examples" mean that specific characteristics, structures, materials, or features described with reference to the embodiment or example are included in at least one embodiment or example of the present disclosure. In this specification, exemplary descriptions of the foregoing terms do not necessarily point at the same embodiment or example. Moreover, the specific characteristics, structures, materials, or features described may be combined in any one or more embodiments or examples in a proper manner.

Although the embodiments of the present disclosure have been shown and described, a person of ordinary skill in the art should understand that various changes, modifications, replacements and variations may be made to the embodiments without departing from the principles and spirit of the present disclosure, and the scope of the present disclosure is as defined by the appended claims and their equivalents.

## Claims

1. A method for screening target cells, the target cells being suitable for secreting target molecules, the method comprising:
culturing candidate single cells in a culture chamber under a condition suitable for secreting the target molecules, wherein the culture chamber includes a signal screening layer for covering the candidate single cells, and wherein the signal screening layer comprises signaling molecules for specific recognition of the target molecules; and
selecting the target cells suitable for secreting the target molecules based on signals of the signaling molecules in the signal screening layer.

2. The method according to claim 1, wherein the signal screening layer is arranged in a matrix material of the culture chamber;
the signal screening layer is arranged in a culture medium;
the signal screening layer comprises at least one kind of antibodies or probes;
the signal screening layer is formed from a hydrogel; or a mechanical strength of the signal screening layer is lower than a mechanical strength of the matrix material.

3. The method according to claim 1 or 2, wherein the culture chamber is arranged on a biological culture chip which comprises:
a matrix formed from a matrix material; and
the culture chamber formed on a surface of the matrix to define the culture chamber.

4. The method according to claim 3, wherein the culture chamber forms a predetermined array pattern on the matrix.

5. The method according to claim 3, wherein an equilibrium swelling ratio of the matrix material of the matrix is approximately 1.25-1.75.

6. The method according to claim 3, wherein the culture chamber has an opening on the surface of the matrix, and the opening is 8-25 µm in diameter and 15-35 µm in depth.

7. The method according to claim 3, wherein the matrix material is selected from the group consisting of collagen hydrogel, methylcellulose, agarose gel and polyacrylamide gel.

8. The method according to claim 3, wherein a distance between at least two adjacent culture chambers is 10-50 µm.

9. The method according to claim 1, wherein the signaling molecules are fluorescent molecules.

10. The method according to claim 9, wherein the fluorescent molecules comprise at least one ofFITC or AF488.

11. The method according to claim 1 or 2, wherein the method further comprises positioning positive cells by a fluorescence microscope.

12. A kit, comprising:
a hydrogel;
a signaling molecule; and
a biological culture chip.

13. A method for preparing antibodies, comprising:
screening for target cells by the method according to any one of claims 1 to 11; and
allowing the target cells to proliferate and express antibodies.

14. A method for screening a monoclonal antibody, comprising:
dispensing a plurality of hybridoma cells onto the biological culture chip according to claim 3, wherein each culture chamber in the biological culture chip contains at most one of the hybridoma cells;
selecting positive hybridoma cells by the method according to any one of claims 1 to 11;
isolating the positive hybridoma cells; and
culturing the positive hybridoma cells to express antibodies, to obtain the monoclonal antibody.

15. A monoclonal antibody, obtained by the method according to claim 14.

16. An antibody-antigen complex, wherein the antibody is the monoclonal antibody according to claim 15, and the monoclonal antibody is linked to a signaling molecule.

17. A method for screening antibodies, comprising:
arranging candidate cells within a culture chamber to be cultured for a predetermined time under a condition suitable for candidate cells to express antibodies;
adding a signaling antibody-antigen complex into the culture chamber, wherein the signaling antibody is linked to a signaling molecule, and the signaling antibody is the monoclonal antibody according to claim 15; and
determining whether the candidate cells are target cells based on signals of the signaling molecules, wherein the target cells secrete antibodies with different antigenic determinants from the monoclonal antibody.

18. A method for screening cells, comprising:
placing candidate cells in a culture chamber for a predetermined time under a condition suitable for candidate cells to express antibodies;
forming a target antibody-antigen-signaling antibody complex in the culture chamber, wherein the signaling antibody is linked to a signaling molecule; and
determining whether the candidate cells are target cells based on signals of the signaling molecules.

19. The method according to claim 18, wherein the predetermined time is 1-10 hours, preferably 2-5 hours, more preferably 3 hours.

20. The method according to claim 18 or 19, wherein the culture chamber is configured on a biological culture chip which comprises:
a matrix formed from the matrix material; and
the culture chamber formed on a surface of the matrix to define the culture chamber;
wherein the target antibody-antigen-signaling antibody complex is formed by adding an antigen-signaling antibody complex into the culture chamber or sequentially adding a free antigen and a free signaling antibody.

21. The method according to claim 20, wherein the culture chamber forms a predetermined array pattern on the matrix.

22. The method according to claim 20, wherein an equilibrium swelling ratio of the matrix material of the matrix is approximately 1.25-1.75.

23. The method according to claim 20, wherein the culture chamber has an opening on the surface of the matrix, and the opening is 8-25 µm in diameter and 15-35 µm in depth.

24. The method according to claim 20, wherein the matrix material is selected from the group consisting of collagen hydrogel, methylcellulose, agarose gel and polyacrylamide gel.

25. The method according to claim 20, wherein a distance between at least two adjacent culture chambers is 10-50 µm.

26. The method according to claim 18 or 19, wherein the signaling molecules are fluorescent molecules, preferably, the fluorescent molecules comprise at least one of FITC or AF488.

27. The method according to claim 18 or 19, wherein the method further comprises positioning positive cells based on the signals of the signaling molecules by a fluorescence microscope.

28. A method for preparing antibodies, comprising:
screening for target cells by the method according to any one of claims 18 to 27; and
allowing the target cells to proliferate and express antibodies to obtain the antibodies with different antigenic determinants from the signaling antibodies.

29. A method for screening for a monoclonal antibody, comprising:
dispensing a plurality of hybridoma cells onto the biological culture chip according to claim 20, wherein each culture chamber in the biological culture chip contains at most one of the hybridoma cells;
selecting positive hybridoma cells by the method according to any one of claims 18 to 27;
isolating the positive hybridoma cells; and
culturing the positive hybridoma cells to express antibodies, to obtain the monoclonal antibody with a different antigenic determinant from the signaling antibody.

30. A monoclonal antibody, obtained by the method according to claim 29.

31. A biological culture chip, comprising:
a matrix formed from a matrix material; and
a culture chamber formed on a surface of the matrix to define the culture chamber.

32. The biological culture chip according to claim 31, wherein an equilibrium swelling ratio of the matrix material of the matrix is approximately 1.25-1.75; and the culture chamber is formed on an opening configured on the surface of the matrix to define the culture chamber.

33. The biological culture chip according to claim 31, wherein the matrix material has a solid-liquid phase transition temperature of 40-45°C.

34. The biological culture chip according to claim 31, wherein the matrix material is capable of being liquid at 37°C.

35. The biological culture chip according to claim 31, wherein during cell growth, a concave is formed on a contact area between the matrix and a cell.

36. The biological culture chip according to claim 31, wherein a depth of the concave is not less than 5% of a diameter of the cell.

37. The biological culture chip according to claim 31, wherein the matrix material is selected from the group consisting of collagen hydrogel, methylcellulose, agarose gel, polyacrylamide gel and mixture of at least two of them in different proportions.

38. The biological culture chip according to claim 31, wherein the matrix further comprises a cytokine corresponding to a biocompatible material.

39. The biological culture chip according to claim 31, wherein the matrix is further selected from the group consisting of fetal bovine serum, protein A/G, collagen, gelatin, bovine serum albumin and mixture of at least two of them.

40. The biological culture chip according to claim 31, wherein a diameter of an opening of the culture chamber is smaller than a diameter of a bottom portion of the culture chamber.

41. The biological culture chip according to claim 40, wherein a diameter of an opening of a microwell is at most 80% of a diameter of a bottom portion of the microwell, preferably 50%.

42. The biological culture chip according to claim 31, wherein a microwell has an opening diameter of 8-25 µm and a depth of 15-35 µm.

43. The biological culture chip according to claim 31, wherein a biocompatible material is B cells, and a diameter of an opening is 8-12 µm; or
a biocompatible material is hybridoma cells, Chinese hamster ovary cells or tumor cells, and a diameter of an opening is 15-25 µm.

44. The biological culture chip according to any one of claims 31 to 43, wherein the culture chamber is configured with a plurality of microwells, the microwells form a predetermined pattern, and a distance between two adjacent microwells is 10-100 µm, preferably 10-50 µm.

45. The biological culture chip according to claim 44, further comprising: a positioning marker formed on the matrix.

46. The biological culture chip according to claim 44, wherein the biocompatible material is hybridoma cells, and a cultural factor of the hybridoma cells is configured on an inner surface of the microwell;
the biocompatible material is MCF10A cells, and insulin is configured on the inner surface of the microwell; or
the biocompatible material is B cells, and at least one or all of CD40L, IL2, or IL10 is configured on the inner surface of the microwell.

47. A template for preparing the biological culture chip according to any one of claims 31 to 46, comprising:
a base plate; and
a microcolumn configured on a surface of the base plate, wherein the microcolumn is adapted for the culture chamber in a form-adapting manner.

48. The template according to claim 47, wherein the microcolumn includes a hydrophobic surface.

49. The template according to claim 47, wherein a surface of the microcolumn is suitable for binding biological factor through a hydrophobic effect.

50. A method for preparing the biological culture chip according to any one of claims 31 to 46, comprising:
applying a liquid biocompatible material to the template according to any one of claims 47 to 49 as a matrix material, and solidifying the matrix material; and
separating the solidified biocompatible material from the template to obtain the biological culture chip.

51. The method according to claim 50, wherein a biological factor or a reagent is pre-adsorbed on a surface of the template.

52. A method for cell culture using the biological culture chip according to any one of claims 31 to 46, comprising:
arranging cells within the culture chamber; and
exposing the biological culture chip to an environment with a predetermined condition.

53. The method according to claim 52, wherein at most one cell is arranged within each culture chamber.
